Europäisches Patentamt

European Patent Office

Office européen des brevets

(19)

(11) EP 0 711 414 B1

(12) FASCICULE DE BREVET EUROPEEN

(45) Date de publication et mention
de la délivrance du brevet:
**10.03.1999 Bulletin 1999/10**

(21) Numéro de dépôt: **94923751.5**

(22) Date de dépôt: **27.07.1994**

(51) Int Cl.⁶: **G01N 33/546**, G01N 33/553,
G01N 33/58

(86) Numéro de dépôt international:
**PCT/FR94/00948**

(87) Numéro de publication internationale:
**WO 95/04279 (09.02.1995 Gazette 1995/07)**

(54) **PROCEDE DE DOSAGE D'UNE SUBSTANCE IMMUNOLOGIQUE AU MOYEN DE PARTICULES DE LATEX MAGNETIQUES ET DE PARTICULES NON-MAGNETIQUES**

VERFAHREN ZUR BESTIMMUNG EINER IMMUNOLOGISCHEN AKTIVEN SUBSTANZ MITTELS MAGNETISCHER LATEXTEILCHEN UND NICHTMAGNETISCHER TEILCHEN

METHOD FOR ASSAYING AN IMMUNOLOGICAL SUBSTANCE USING MAGNETIC LATEX PARTICLES AND NON-MAGNETIC PARTICLES

(84) Etats contractants désignés:
**AT BE DE ES FR IT NL SE**

(30) Priorité: **28.07.1993 FR 9309296**

(43) Date de publication de la demande:
**15.05.1996 Bulletin 1996/20**

(73) Titulaires:
• **SOCIETE DIAGNOSTICA-STAGO**
  **F-92600 Asnières (FR)**
• **ALFA BIOTECH SpA**
  **00040 Pomezia (Roma) (IT)**

(72) Inventeurs:
• **ESTEVE, Frédéric**
  **F-75003 Paris (FR)**
• **AMIRAL, Jean**
  **F-95130 Franconville (FR)**

• **PADULA, Cristiano**
  **I-00135 Rome (IT)**
• **SOLINAS, Isabella**
  **I-00199 Rome (IT)**

(74) Mandataire: **Clisci, Serge**
  **S.A. FEDIT-LORIOT**
  **CONSEILS EN PROPRIETE INDUSTRIELLE**
  **38, avenue Hoche**
  **75008 Paris (FR)**

(56) Documents cités:
EP-A- 0 310 872          EP-A- 0 410 893
WO-A-86/06493          US-A- 4 115 535

• **DATABASE WPI Section Ch, Week 9334,
Derwent Publications Ltd., London, GB; Class
B04, AN 93267053 & JP,A,5 180 842 (NIPPON
PAINT CO LTD) 23 Juillet 1993 cité dans la
demande**

## Description

### *Domaine de l'invention*

La présente invention a trait à un nouveau procédé de dosage d'une substance immunologique choisie parmi l'ensemble constitué par les antigènes et les anticorps, ledit procédé mettant en oeuvre des particules de latex magnétiques servant de "support" à la réaction immunologique

antigène + anticorps → antigène-anticorps

et des particules non-magnétiques servant de "marqueur" dans ladite réaction.

Elle concerne également, en tant que produits industriels nouveaux, les trousses ou nécessaires de dosage comprenant lesdites particules magnétiques et non-magnétiques sensibilisées avec des composants (ou substances) immunologiques.

### Art antérieur

Historiquement, on sait que des techniques de dosage reposant sur l'agglutination macroscopique (i.e. agglutination visible à l'oeil nu) de particules de latex sensibilisées par un antigène (ou respectivement un anticorps) par mise en contact avec un conjugué anticorps (ou respectivement antigène) ont été présentées pour la première fois dans l'article de J.M. SINGER, American Journal of Medecine, 31, pages 766-779, (1961).

Ces techniques de dosage ont été améliorées, en ce qui concerne la sensibilité et la durée de réaction, en remplaçant la détection à l'échelle macroscopique de l'agglutination, par une détection à l'échelle microscopique de la réaction d'agglutination antigène/anticorps au moyen d'une variation de l'intensité lumineuse transmise ou diffractée (i.e. diffusée). Ainsi ont été proposés des dosages quantitatifs ou qualitatifs par turbidimétrie ou par néphélométrie.

Dans le domaine de la turbidimétrie (par mesure de l'absorbance de la lumière), on connaît des articles de A.M. BERNARD et al., Clin. Chem., 27 (No. 6), pages 832-837, (1981) et de A.M. BERNARD et R.R. LAUWERYS, Clin. Chem. Acta, 119, pages 335-339, (1982), l'utilisation de particules submicroniques de latex (diamètre moyen : 0,79 micromètre) revêtues d'anticorps anti ($\beta_2$-microglobuline) par mesure de l'absorbance à 360 nm ; de l'article de Y. MAYNARD et al., Clin. Chem., 32 (No. 5), pages 752-757, (1986), l'utilisation d'anticorps monoclonaux ou polyclonaux anti-IgG pour le dosage, dans les microcuvettes, d'immunoglobulines sériques.

Dans le domaine de la néphélométrie (par mesure de la diffusion de la lumière) on connaît de l'article de J. GRANGE et al., Journal of Immunological Methods, 18, pages 365-375, (1977) l'utilisation de billes de latex submicroniques (diamètre moyen : 0,3 micromètre) revêtues d'un anticorps, anti-IgG de lapin, pour le dosage d'IgG de lapin. Les billes de latex sont ici constituées par du polystyrène carboxylé contenant les groupes COOH pour fixer le ligand ou composant immunologique, dans le cas d'espèce l'anticorps anti-IgG de lapin. Cet article signale notamment que "la préparation de sphères de latex contenant des antigènes ou des anticorps adsorbés est difficile à standardiser en raison de leur tendance à s'auto-agglutiner lorsque le pH et la force ionique du milieu de dosage changent".

On connaît également de l'article d'Anne-Marie BONNEFOY et al., C.R. Acad. Sc. Paris, 283, série D, pages 115-118 (5 juillet 1976) l'utilisation de sphères de latex (polystyrène) d'un diamètre de 300 nm liées par covalence à un anticorps pour la réalisation de dosages néphélométriques (lecture sous une longueur d'onde de 550 nm, selon un angle d'observation de 90°).

En bref, les méthodes turbidimétriques et néphélométriques, qui sont utilisables pour des concentrations de substance immunologique supérieures à 0,1 mg/ml (méthodes turbidimétriques) ou supérieures à 0,02 mg/ml (méthodes néphélométriques), requièrent un matériel très "spécialisé" et un traitement préalable des échantillons. Elles sont limitées par plusieurs inconvénients à savoir :

-   difficultés à réaliser des suspensions stables et standardisées,
-   auto-agglutination spontanée des latex sensibilisés par un anticorps ou un antigène,
-   faible plage de mesure de la courbe d'étalonnage (variation de la densité optique (OD) n'excédant pas 0.3 à 0.4),
-   manque de sensibilité et de reproductibilité,
-   traitement du réactif particulaire avant emploi dans certains cas (pour désagrégation), et mauvaise stabilité du réactif,
-   mise en oeuvre délicate et détection parfois complexe.

Ces inconvénients ont fait que ces méthodes n'ont pas été exploitées malgré leur intérêt pratique.

On connaît par ailleurs de US-A-5 175 112 (J. AMIRAL et al.) un procédé de dosage turbidimétrique ou néphélométrique par agglutination d'un réactif constitué par des particules de latex submicroniques sensibilisées par un anticorps (ou respectivement un antigène) en présence de l'antigène correspondant à tester, ce procédé permettant d'améliorer la sensibilité (seuil de sensibilité de l'ordre de 100 ng/ml par turbidimétrie, et de l'ordre de 10 ng/ml par néphélométrie) par un choix particulier de la nature du latex, de la granulométrie des particules dudit latex, du mode de stabilisation desdites particules, de la force ionique et du pH du milieu réactionnel liquide.

Par ailleurs, on connaît des trousses ou nécessaires de dosage comprenant des particules de latex magnétiques et plus particulièrement des particules de latex paramagnétiques (les fabricants de particules de latex les appellent notamment des particules de latex "superparamagnétiques"). Il se trouve que les protocoles de dosage fournis avec ces trousses (notamment celui des trousses commercialisées par les sociétés dites AMERSHAM et CIBA-CORNING) mettent en oeuvre un procédé différent de celui de l'invention, en ce sens que lesdites particules de latex magnétiques sensibilisées par un matériau immunologique interviennent principalement pour remplacer, dans une méthode EIA ("enzymoimmunoassay") ou RIA ("radioimmunoassay"), une opération de filtration ou centrifugation. En particulier les protocoles de dosage des trousses connues contenant des particules de latex magnétiques (i) ne concernent pas le domaine de l'agglutination, et (ni) ne décrivent ni ne suggèrent en particulier le procédé préféré selon l'invention dit de dosage "en retour" direct ou en un seul temps (c'est-à-dire en opérant dans le même milieu et le même récipient de réaction, sans rinçage ni apport supplémentaire de réactif ou diluant), d'une part, ni le dosage "direct" (inverse du dosage dit en "retour") par inhibition compétitive en un seul temps, d'autre part.

Par ailleurs, on connaît de US-A-4 115 535 (I. GIAEVER), JP-A-5 180 842 (NIPPON PAINT Co. Ltd.) [résumé dans DATABASE WPI de DERWENT, No. 93-267053 et publié le 23.07.1993] et de WO-A-8606493 (LABSYSTEMS OY) une technique de dosage/identification selon laquelle on fait

> (1) réagir (a) des particules sensibilisées par le même antigène, ces particules étant les unes magnétiques les autres non-magnétiques et colorées, avec (b) un échantillon susceptible de contenir un anticorps spécifique dudit antigène, et
> (2) appliquer un champ magnétique pour observer dans la portion non-agglutinée du milieu réactionnel liquide, une diminution de la densité optique traduisant la présence dudit anticorps dans ledit échantillon.

Selon le résumé de JP-A-5 180 842 précité, on peut également faire réagir (a) des particules magnétiques et non-magnétiques sensibilisées par le même anticorps avec (b) un échantillon susceptible de contenir l'antigène correspondant.

Cette technique de compétition/agglutination présente cependant l'inconvénient de ne pas être suffisamment sensible par rapport aux méthodes EIA et RIA connues.

On connaît enfin de EP-A-0 410 893 (MITSUBISHI KASEI) une technique de dosage (ou identification) d'un anticorps susceptible d'être présent dans un échantillon de fluide biologique, selon laquelle

> (1) on fait réagir ledit échantillon avec un mélange comprenant (a) des particules non-magnétiques sensibilisées par l'antigène spécifique dudit anticorps, et (b) des particules magnétiques sensibilisées par un produit fixant les anticorps [tel que la protéine A, une autre substance fixant le fragment Fc des anticorps ou un anti(anticorps)],
> (2) on applique un champ magnétique pour séparer les particules non-magnétiques qui n'ont pas été agglutinées avec les particules magnétiques, et
> (3) on évalue la quantité de particules non-magnétiques qui n'ont pas été agglutinées pour en déduire la quantité d'anticorps présente dans ledit échantillon.

Dans cette dernière technique, les particules magnétiques sensibilisées par le produit fixant les anticorps interviennent comme un moyen amplificateur de la réaction de l'antigène avec l'anticorps que l'on veut déterminer. Cependant cette technique présente l'inconvénient de ne pas être suffisamment sensible par rapport aux méthodes EIA et RIA connues.

### But de l'invention

Un des buts de l'invention est de proposer une nouvelle solution technique pour le dosage, la détermination ou l'identification par agglutination à l'échelle microscopique, qui soit essentiellement plus rapide et subsidiairement plus sensible que les méthodes antérieurement connues de dosage d'agglutination par turbidimétrie, néphélométrie ou champ magnétique.

L'objectif idéal, en ce qui concerne la sensibilité, serait d'atteindre, par une technique d'agglutination ou par une technique homogène en un seul temps, ne nécessitant pas les lavages habituels des techniques EIA, le seuil (concentration minimale de la substance immunologique à doser se situant dans la gamme de 10 ng/ml à 1 ng/ml) des techniques EIA ou RIA.

Un autre but de l'invention est de pallier aux inconvénients précités des techniques de dosage de l'agglutination par turbidimétrie, néphélométrie ou champ magnétique.

Selon l'invention est envisagée pour le dosage ou l'identification d'une substance immunologique [X], une nouvelle solution technique mettant en oeuvre des particules magnétiques et non-magnétiques qui sont (i) les unes sensibilisées par un anticorps [antiX] de ladite substance immunologique, et (ni) les autres sensibilisées par ladite substance immunologique [X], un anticorps [anti(antiX)] dudit anticorps [antiX] ou un anticorps identique ou différent dudit anticorps [antiX].

### Objet de l'invention

Ces buts sont atteints grâce à la nouvelle solution technique selon l'invention permettant de détecter la portion non agglutinée (avec un photomètre de laboratoire relativement simple) d'un système comprenant un réactif immunologique constitué par des particules de latex magnétiques sensibilisées par un premier composant immunologique, d'une part, et un réactif immunologique constitué par des particules non-magnétiques sensibilisées par un second composant immunologique, d'autre part, l'un desdits premier et second composants étant un anticorps de la substance immunologique X à tester, l'autre étant anti(X), anti(antiX) ou ladite substance immunologique X.

Selon un premier aspect de l'invention, on préconise donc un procédé de dosage, d'identification ou de détermination en retour de façon directe d'une substance immunologique (X) choisie parmi l'ensemble constitué par les antigènes et les anticorps et susceptible d'être présente dans un échantillon à tester, ledit procédé, qui met en oeuvre (i) des particules de latex magnétiques, (ni) des particules non-magnétiques et (iii) une réaction immunologique

$$X + antiX \rightarrow X(antiX),$$

étant caractérisé en ce qu'il comprend les étapes consistant à

(1°) mettre en contact dans un milieu liquide ledit échantillon à tester et pouvant contenir ladite substance immunologique avec des quantités biologiquement connues

(A) d'un premier réactif immunologique en suspension dans un tampon et constitué par des particules de latex magnétiques sensibilisées par un premier composant qui est un anticorps (antiX) de ladite substance immunologique, et
(B) d'un second réactif immunologique en suspension dans un tampon et constitué par des particules non-magnétiques sensibilisées par un second composant qui est un anticorps de ladite substance immunologique, ledit second composant étant (i) stoechiométriquement en excès par rapport à ladite substance immunologique X à tester et (ii) différent dudit premier composant (antiX) ;

(2°) incuber le milieu réactionnel résultant pendant une durée inférieure ou égale à 1 h pour mettre en oeuvre la ou les réactions immunologiques X + antiX → X-(antiX) ;
(3°) appliquer un champ magnétique pour séparer, dans le milieu réactionnel résultant, les produits magnétiques déplacés par ledit champ magnétique des produits non-magnétiques non affectés par ledit champ magnétique ; puis,
(4°) évaluer ladite substance immunologique (X), pouvant être présente dans l'échantillon à tester, par l'observation de la portion (P) du milieu réactionnel résultant ne contenant que des produits non-magnétiques en suspension dans ladite portion (P) et non affectés par la réaction immunologique et ledit champ magnétique.

Selon un second aspect de l'invention on préconise un procédé de dosage, d'identification ou de détermination en retour de façon directe d'une substance immunologique (X) choisie parmi l'ensemble constitué par les anticorps et susceptible d'être présente dans un échantillon à tester, ledit procédé, qui met en oeuvre (i) des particules de latex magnétiques, (ni) des particules non-magnétiques et (iii) une réaction immunologique

$$Antigène + anticorps \rightarrow antigène - anticorps$$

étant caractérisé en ce qu'il comprend les étapes consistant à

(1°) mettre en contact dans un milieu liquide ledit échantillon à tester et pouvant contenir ladite substance immunologique avec des quantités biologiquement connues

(A) d'un premier réactif immunologique en suspension dans un tampon et constitué par des particules de latex magnétiques sensibilisées par un premier composant qui est un antigène spécifique de ladite substance immunologique, et
(B) d'un second réactif immunologique en suspension dans un tampon et constitué par des particules non-magnétiques sensibilisées par un second composant qui est un antigène de ladite substance immunologique, ledit second composant étant (i) stoechiométriquement en excès par rapport à ladite substance immunologique X à tester et (ni) différent dudit premier composant antigène ;

(2°) incuber le milieu réactionnel résultant pendant une durée inférieure ou égale à 1 h pour mettre en oeuvre la ou les réactions immunologiques antigène + anticorps → antigène-anticorps ;
(3°) appliquer un champ magnétique pour séparer, dans le milieu réactionnel résultant, les produits magnétiques déplacés par ledit champ magnétique des produits non-magnétiques non affectés par ledit champ magnétique ; puis,
(4°) évaluer ladite substance immunologique (X), pouvant être présente dans l'échantillon à tester, par l'observation de la portion (P) du milieu réactionnel résultant ne contenant que des produits non-magnétiques en suspension dans ladite portion (P) et non affectés par la réaction immunologique et ledit champ magnétique.

Selon un troisième aspect de l'invention, on préconise un procédé de dosage, d'identification ou de détermination d'une substance immunologique (X) choisie parmi l'ensemble constitué par les antigènes et les anticorps et susceptible d'être présente dans un échantillon à tester, ledit procédé, qui met en oeuvre (i) des particules de latex magnétiques, (ni) des particules non-magnétiques et (iii) une réaction immunologique

$$X + antiX \rightarrow X\text{-}(antiX),$$

étant caractérisé en ce qu'il comprend les étapes consistant à

(1°) mettre en contact dans un milieu liquide ledit échantillon à tester et pouvant contenir ladite substance immunologique avec des quantités biologiquement connues

(A) d'un premier réactif immunologique en suspension dans un tampon et constitué par des particules de latex magnétiques sensibilisées par un premier composant qui est un anticorps (antiX) de ladite substance immunologique, et
(B) d'un second réactif immunologique en suspension dans un tampon et constitué par des particules non-magnétiques sensibilisées par un second composant qui est ladite substance immunologique ;

(2°) incuber le milieu réactionnel résultant pendant une durée inférieure ou égale à 1 h pour mettre en oeuvre la ou les réactions immunologiques $X + antiX \rightarrow X\text{-}(antiX)$ ;
(3°) appliquer un champ magnétique pour séparer, dans le milieu réactionnel résultant, les produits magnétiques déplacés par ledit champ magnétique des produits non-magnétiques non affectés par ledit champ magnétique ; puis,
(4°) évaluer ladite substance immunologique (X) à tester, pouvant être présente dans l'échantillon à tester, par l'observation de la portion (P) du milieu réactionnel résultant ne contenant que des produits non-magnétiques en suspensions dans ladite portion (P) et non affectés par la réaction immunologique et ledit champ magnétique.

Selon l'invention on propose également un nécessaire, kit ou trousse de dosage pour doser, identifier ou déterminer une substance immunologique (X) pouvant être contenue dans un échantillon à tester, ledit nécessaire étant caractérisé en ce qu'il comprend

(A) un réactif immunologique constitué par des particules de latex magnétiques sensibilisées par ledit premier composant, et
(B) un réactif immunologique constitué par des particules non-magnétiques sensibilisées par ledit second composant.

Plus précisément, ledit nécessaire comprend

- pour la mise en oeuvre du procédé selon le premier aspect ci-dessus :

(A) un réactif immunologique constitué par des particules de latex magnétiques sensibilisées par un premier anticorps (antiX) de la substance X, et
(B) un réactif immunologique constitué par des particules non-magnétiques sensibilisées par un second anticorps de la substance X différent dudit premier anticorps antiX ;

- pour la mise en oeuvre du procédé selon le second aspect ci-dessus où la substance immunologique X à tester est un anticorps :

(A) un réactif immunologique constitué par des particules de latex magnétiques sensibilisées par un premier antigène spécifique de l'anticorps X, et
(B) un réactif immunologique constitué par des particules non-magnétiques sensibilisées par un second antigène spécifique de l'anticorps X et différent dudit premier antigène ; et,

- pour la mise en oeuvre du procédé selon le troisième aspect ci-dessus :

(A) un réactif immunologique constitué par des particules de latex magnétiques sensibilisées par un premier composant qui est un anticorps (antiX) de la substance X, et
(B) un réactif immunologique constitué par des particules non-magnétiques sensibilisées par un second composant qui est ladite substance X.

### Abréviations

Par commodité les abréviations suivantes ont été utilisées dans la présente invention.

CMV  désigne un cytomégalovirus ;
D  désigne un fragment monomère particulier appartenant à l'ensemble de FDP ;
DDi  désigne un fragment D dimère particulier appartenant à l'ensemble des FnDP ;
EIA  désigne un essai enzymo-immunologique (de l'anglais : "enzymoimmunoassay") ;
F(ab)  désigne un fragment d'un anticorps du type 1g comportant les branches a et b et obtenu par clivage dudit anticorps par la papaïne ;
F(ab')$_2$  désigne un fragment d'un anticorps du type Ig comportant les branches a et b et obtenu par clivage dudit anticorps par la pepsine ;
Fc  désigne un fragment d'un anticorps du type Ig essentiellement constitué par la branche c et obtenu par clivage chimique ou enzymatique ; les fragments Fc sont homologues mais structurellement différents selon le mode de clivage [voir à cet effet les indications fournies dans la publication FR-A-2 645 647 en ce qui concerne les Fc séparés de F(ab) et F(ab')$_2$] ;

FDP désigne les produits de dégradations du type FgDP ou FnDP ;

FgDP désigne les produits de dégradation du fibrinogène ;

FnDP désigne les produits de dégradation de la fibrine ;

GMV désigne un virus de la rubéole (de l'anglais : "German measles virus") ;

HBcAb désigne un anticorps de noyau du virus de l'hépatite B (en anglais : "hepatitis B core antibody") ;

HBcAg désigne un antigène de noyau du virus de l'hépatite B (en anglais : "hepatitis B core antigen") ;

HBsAb désigne un anticorps de surface du virus de l'hépatite B (en anglais : "hepatitis B surface antibody") ;

HBsAg désigne un antigène de surface du virus de l'hépatite B (en anglais : "hepatitis B surface antigen") ;

HCV désigne un virus de l'hépatite C ;

HIV désigne un virus d'immunodéficience humaine (en anglais : "human immunodeficiency virus") ;

HSV désigne un virus de l'herpès (en anglais : "herpes simplex virus");

Ig désigne une immunoglobuline, notamment une IgG, une IgM ou un mélange (IgG/IgM) des deux ;

IV$_A$ désigne un virus de la grippe de type A (en anglais : "influenza A virus") ;

IV$_B$ désigne un virus de la grippe de type B (en anglais : "influenza B virus") ;

OD désigne la densité optique ;

PAI désigne un inhibiteur des activateurs du plasminogène ;

PAI-1 désigne l'inhibiteur 1 des activateurs du plasminogène, substance formant des complexes (tPA-PAI-1) et (uPA-PAI-1) avec tPA et respectivement uPA ;

RF désigne le facteur rhumatoïde ;

RIA désigne un essai radio-immunologique (de l'anglais : "radioimmunoassay") ;

RSV désigne un virus de la syncinésie respiratoire (en anglais "respiratory syncitial virus") ;

RT désigne la température ambiante (18-25°C) ;

tPA désigne tout activateur du plasminogène de type tissulaire (en anglais : "tissue plasminogen activator") et comprend les sctPA (activateur du plasminogène de type tissulaire de structure monocaténaire, en anglais : "single-chain tissue plasminogen activator") et tctPA (activateur du plasminogène de type tissulaire de structure bicaténaire, en anglais : "two-chain tissue plasminogen activator") ;

TV désigne un virus de la toxoplasmose ;

uPA désigne tout activateur du plasminogène de type urokinase (de l'anglais : "urokinase plasminogen activator") et comprend les scuPA (activateur du plasminogène de type urokinase de structure monocaténaire, en anglais : "single-chain urokinase plasminogen activator", également appelé prourokinase) et tcuPA (activateur du plasminogène de type urokinase de structure bicaténaire, en anglais : "two-chain urokinase plasminogen activator", également dénommé urokinase) ;

9C3 anticorps monoclonal spécifique du DDi (commercialisé par la société dite DIAGNOSTICA STAGO) ;

2F7 anticorps monoclonal spécifique du DDi (commercialisé par la société dite DIAGNOSTICA STAGO) ;

7D4 anticorps monoclonal spécifique du PAI-1 (commercialisé par la société dite DIAGNOSTICA STAGO) ;

7F5 anticorps monoclonal spécifique du PAI-1 (commercialisé par la société dite DIAGNOSTICA STAGO).

**Brève description des dessins**

Dans les dessins annexés, nullement limitatifs,

- la figure 1 illustre schématiquement les mécanismes réactionnels d'un dosage selon l'invention, dit "en retour" et utilisant des particules de latex magnétiques sensibilisées par un anticorps levé contre la substance immunologique à doser et des particules de latex non-magnétiques sensibilisées par un anticorps levé contre ladite substance immunologique ;

- la figure 2 montre une courbe "dose-réponse" établie par turbidimètrie (OD mesurée à 450 nm) et relative à l'étalonnage du dosage du DDi selon l'invention ;

- la figure 3 montre une courbe "dose-réponse" établie, par comparaison, dans les mêmes conditions que la courbe de la figure 2, selon les modalités opératoires EIA fournies dans la notice de la trousse "ASSERACHROM[R] D-DIMERE" (commercialisée par la société dite DIAGNOSTICA STAGO pour le dosage du DDi) ;

- la figure 4 montre la corrélation entre les dosages du DDi selon le procédé de l'invention (figure 2) et selon la méthode EIA (figure 3) ;

- la figure 5 montre la courbe "dose-réponse" du dosage de la Protéine C selon l'invention ;
- la figure 6 montre une courbe "dose-réponse" établie par fluorescence et relative à l'étalonnage du dosage direct du DDi selon l'invention ;
- la figure 7 montre une courbe "dose-réponse" établie par fluorescence et relative à l'étalonnage du dosage en retour du PAI-1 selon l'invention ; et,
- la figure 8 montre une courbe "dose-réponse" établie par turbidimétrie (OD mesurée à 450 nm) et relative à l'étalonnage du dosage en retour de HBsAb selon le procédé de l'invention.

### Description détaillée de l'invention

Par l'expression "substance immunologique" on entend ici tout élément d'un couple (antigène/anticorps) intervenant dans une réaction immunologique :

antigène + anticorps → antigène-anticorps

Par "substances antigéniques" on entend ici les antigènes proprement dits, d'une part, et les substances à partir desquelles on peut générer des anticorps, d'autre part. Parmi les substances à partir desquelles on peut générer les anticorps, on peut notamment citer les haptènes, les peptides, les médicaments comportant au moins un fragment peptidique, les alcaloïdes et d'une manière générale toutes substances présentant une structure immunologique.

Le premier composant immunologique est fixé, par (i) adsorption ou (ni) par covalence (i.e. par l'intermédiaire d'un composé bifonctionnel formant un pont), sur des particules de latex magnétiques.

Les modalités de fixation par adsorption ou covalence sont décrites dans le brevet US-A-5 175 112 précité incorporé ici à titre de référence en ce qui concerne (i) la fixation par adsorption et par covalence, (ni) la stabilisation des particules de latex et (iii) le nombre de milliéquivalents de groupes COOH par gramme de latex.

Les particules de latex de ce premier réactif immunologique sont magnétiques, à savoir diamagnétiques (elles sont repoussées par un aimant) ou mieux selon l'invention paramagnétiques (elles sont attirées par un aimant). Ces particules de latex sont sensiblement sphériques et se présentent avantageusement sous la forme de microbilles ayant un diamètre se situant dans l'intervalle 0,02 - 20,0 micromètres.

Le second composant immunologique est fixé sur des particules non-magnétiques capables de fixer un antigène ou un anticorps, ces particules non-magnétiques étant notamment choisies parmi l'ensemble constitué par les particules de latex, les particules métalliques et les autres particules adsorbantes telles que les argiles (notamment la bentonite) et le charbon actif.

Les particules de latex non-magnétiques peuvent être soit non-colorées, soit colorées, soit encore susceptibles de fournir une coloration. L'utilisation de particules de latex non-magnétiques fluorescentes, phosphorescentes, luminescentes, ou chemiluminescentes améliore la sensibilité du dosage selon l'invention. Les particules métalliques peuvent être notamment des particules colloïdales d'or ou d'argent.

La fixation du second composant immunologique sur des particules de latex non-magnétiques est réalisée par adsorption ou par covalence comme indiqué ci-dessus ; la fixation du second composant sur des particules métalliques (telles que Au colloïdal ou Ag colloïdal) ou les autres particules adsorbantes (telles que la bentonite et le charbon actif) est en général réalisée par adsorption. Les particules non-magnétiques, (avantageusement les particules de latex non-magnétiques), servant à l'élaboration du réactif immunologique contenant ledit second composant immunologique, sont sensiblement sphériques et se présentent avantageusement sous la forme de microbilles ayant un diamètre se situant dans l'intervalle 0,01 - 10,0 micromètres.

De façon encore plus avantageuse, les particules de latex magnétiques (de préférence ici des particules de latex paramagnétiques) et les particules non-magnétiques (notamment les particules de latex non magnétiques) seront des particules submicroniques, c'est-à-dire des microbilles ayant un diamètre inférieur ou égal à 1 micromètre, même si des particules d'un diamètre de 1 à 20 micromètres donnent des résultats corrects.

On recommande plus particulièrement d'utiliser selon l'invention (i) des particules de latex magnétiques qui sont sensibilisées par le premier composant immunologique et qui ont un diamètre compris dans l'intervalle de 300 - 800 nm, et (ii) des particules non-magnétiques qui sont sensibilisées par le second composant immunologique et qui ont un diamètre compris dans l'intervalle de 100 - 600 nm.

Les latex utilisés dans la mise en oeuvre de l'invention seront avantageusement des latex décrits dans US-A-5 175 112 précité ou encore des latex chlorométhylés.

Le procédé de l'invention permet l'identification, dans un milieu biologique aqueux tel que le sang (plasma ou sérum), urine, salive ou lait, d'une substance immunologique qui peut être (i) une substance antigénique telle que DDi, PAI, tPA (notamment sctPA et/ou tctPA), uPA (notamment scuPA et/ou tcuPA), Protéine C, (ii) un antigène d'origine bactérienne ou virale tel que HBcAg, HBsAg, antigène de IV$_A$ ou antigène de IV$_B$, ou (iii) un anticorps présent dans ou dirigé contre une portion d'enveloppe ou noyau de bactérie, moisissure ou virus tel que HBcAb, HBsAb, anticorps de CMV, GMV, HCV, HIV, HSV, MCV, RCV ou TV.

En pratique, selon une première solution technique, la substance immunologique (X) à doser sera une substance antigénique (notamment DDi, FgDP, FnDP, PAI, Protéine C, HBcAb, HBsAg, RSV, $IV_A$, $IV_B$, adénovirus, etc...), le premier réactif immunologique sera alors sensibilisé par un composant anticorps (antiX) spécifique de ladite substance immunologique, et le second réactif immunologique sera sensibilisé par un composant anticorps monoclonal spécifique de ladite substance immunologique, différent de l'anticorps monoclonal (antiX) du premier réactif immunologique.

En pratique, selon une seconde solution technique, la substance immunologique (X) à doser sera un anticorps (notamment HBsAb, HBcAb (Ig totale/IgM), un anticorps IgG/IgM de TV, un anticorps IgG/IgM de GMV, un anticorps IgG/IgM de CMV, un anticorps IgG/IgM de HSV, un anticorps IgG de HCV, un anticorps IgG de HIV, etc...) et

> (i) le premier matériau immunologique sera alors sensibilisé par un composant anticorps [antiX, i.e. un anti(anticorps)] de ladite substance immunologique X et le second réactif immunologique sera sensibilisé par un composant antigène spécifique de ladite substance immunologique X, ou
> (ii) le premier réactif immunologique et le second réactif immunologique seront sensibilisés chacun par un antigène différent et spécifique de ladite substance immunologique.

Dans la réaction immunologique d'agglutination selon le procédé de l'invention les particules de latex magnétiques interviennent en tant que "support" et les particules non-magnétiques en tant que "marqueur". Après incubation ou réaction (selon le stade (2°) ci-dessus), sous l'action d'un champ magnétique, notamment créé par un aimant, toutes les particules de latex magnétiques (celles qui ont réagi et celles qui n'ont pas réagi) sont rassemblées et entraînent donc les produits réactionnels résultant de l'agglutination, d'une part, tandis que les particules non-magnétiques libres, c'est-à-dire non agglutinées avec lesdites particules de latex magnétiques soit directement (quand le second composant immunologique est la substance immunologique X) soit par l'intermédiaire de la substance antigénique à doser (quand le second composant immunologique est un anticorps antiX de la substance immunologique X à tester), restent en suspension et permettent une évaluation soit à l'oeil nu soit au moyen d'un photomètre, d'autre part.

L'évaluation de l'étape (4°) est effectuée par la mesure de la variation de densité optique (OD), ladite variation étant (i) proportionnelle à la concentration en particules non-magnétiques non affectées par le champ magnétique dans ladite portion (P) et (ni) inversement proportionnelle à la concentration en substance X à tester.

En pratique, cette évaluation est effectuée par une variation de OD notamment par la détermination de l'absorbance ou de la diffusion de la lumière, soit encore par fluorescence, phosphorescence, luminescence ou chemiluminescence.

Ainsi, quand la substance X à tester est un antigène ou un anticorps et les premier et second composants immunologiques sont des anticorps monoclonaux (voire même polyclonaux) spécifiques de ladite substance X utilisés selon des quantités connues (ledit second composant immunologique étant stoechiométriquement en excès par rapport à ladite substance X), on réalise un dosage en retour de façon directe car après séparation au moyen du champ magnétique la variation de OD est (i) proportionnelle à la quantité de particules non-magnétiques sensibilisées par le second composant immunologique restant en suspension et (ii) inversement proportionnelle à (ou une fonction décroissante de) la concentration en substance X à tester.

En revanche si la substance X à tester est un antigène, le premier composant immunologique est un anticorps monoclonal (antiX) spécifique de ladite substance X (utilisé selon une quantité connue) et le second composant immunologique est la substance X (utilisée selon une quantité connue et marquée) ou un anticorps monoclonal [anti(antiX)] dirigé contre antiX (utilisé selon une quantité connue et marqué), on réalise alors après séparation au moyen du champ magnétique un dosage en direct (qui est un dosage inverse du dosage dit en retour) de l'antigène X à doser.

Le marquage du réactif immunologique constitué par des particules sensibilisées par le second composant immunologique concerne ici soit ledit second composant (marquage notamment par couplage avec un moyen fluorescent), soit lesdites particules (marquage résultant notamment de l'utilisation de particules colorées ou fluorescentes).

De plus, comme indiqué ci-dessus l'utilisation de particules colorées ou fluorescentes pour les particules sensibilisées par le second composant immunologique, notamment des microbilles de latex colorées, fluorescentes, phosphorescentes, luminescentes ou chemiluminescentes améliore la sensibilité du dosage selon le procédé de l'invention.

Comme les milieux biologiques liquides contenant la substance immunologique à tester peuvent contenir le RF, notamment dans le cadre d'un dosage sur plasma d'un produit intervenant dans les mécanismes de l'hémostase, il est parfois utile et avantageux de remplacer l'anticorps des premier et second composants immunologiques par l'un des fragments (dépourvu de la branche c sensible audit RF) tels que F(ab) ou mieux F$(ab')_2$.

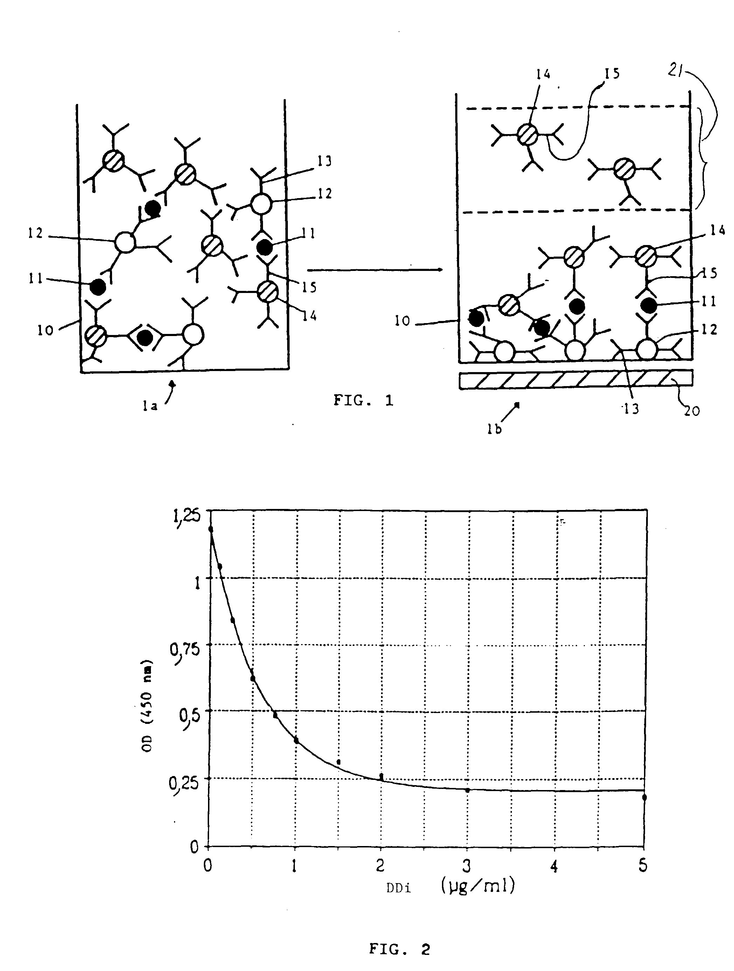

La figure 1 illustre schématiquement les mécanismes de mise en oeuvre d'un dosage en retour de façon directe. Selon la portion 1a, on met en contact et laisse réagir (pendant une durée inférieure ou égale à 1 h, de préférence pendant une durée inférieure ou égale à 0, 75 h, et mieux pendant une durée de 5 à 30 minutes ; on arrive dans la majorité des cas à une durée de dosage de 10-15 minutes voire inférieure à 10 minutes) dans un réacteur (ici une cuvette ou microcuvette) une substance immunologique 11, à doser qui est un antigène, des particules de latex paramagnétiques 12 sensibilisées par un premier anticorps monoclonal 13 dirigé spécifiquement contre ladite substance immunologique, et des particules de latex non-magnétiques 14 (de préférence colorées ou fluorescentes) sensibilisées par un second anticorps monoclonal 15 dirigé spécifiquement contre ladite substance immunologique.

Après incubation (i.e. réaction) à une température appropriée (notamment comprise entre RT et 60°C, et mieux à RT) on applique, selon la portion 1b, un champ magnétique en approchant un aimant 20 au voisinage du fond du récipient réactionnel 10. Les produits réactionnels résultant de l'agglutination

$$11+(12\text{-}13)+(14\text{-}15)\rightarrow(12\text{-}13)\text{-}11\text{-}(14\text{-}15)$$

et le cas échéant le réactif immunologique (12-13) qui n'aurait pas réagi sont attirés par l'aimant 20, tandis que l'excès du réactif (14-15) qui n'a pas réagi reste en suspension. En d'autres termes, sous l'action du champ magnétique, les particules de latex magnétiques entraînent et rassemblent les produits réactionnels qui résultent de l'agglutination, alors que les particules non-magnétiques en excès et non incluses dans lesdits produits réactionnels permettent une mesure de OD par l'intermédiaire d'une portion (P) transparente 21 prévue sur la paroi latérale du récipient réactionnel 10, puis d'en déduire la concentration de ladite substance immunologique contenue dans l'échantillon à doser. Cette concentration peut être déterminée à partir d'une courbe d'étalonnage ou encore, le cas échéant, à partir d'un ou deux essais de contrôle. Pendant la mesure de OD, on maintient le champ magnétique.

D'une manière générale, une durée d'incubation de 5 minutes à 30 minutes est suffisante pour permettre une évaluation très précise : on arrive à un seuil de sensibilité de l'ordre de 1-10 ng/ml, notamment pour le dosage de la Protéine C, identique ou analogue au seuil inférieur (1 ng/ml) des techniques EIA et RIA actuellement utilisées. Dans le dosage du PAI-1, avec des latex fluorescents, on obtient également une sensibilité identique voire même inférieure au seuil desdites techniques EIA et RIA.

De façon similaire, le dosage "en retour" de la figure 1, peut être modifié en dosage "direct" en remplaçant l'anticorps monoclonal 15 fixé sur des particules 14, par la substance immunologique utilisée selon une quantité connue et fixée sur des particules 14 appropriées. On a alors un dosage dans lequel les antigènes 11 (la substance X à tester) et 15 (ainsi modifié) réagissent en compétition avec l'anticorps 13 fixé sur les particules de latex paramagnétiques 12.

L'intérêt du procédé selon l'invention réside essentiellement dans la rapidité et la simplicité de sa mise en oeuvre au sein d'un même milieu liquide dans le même récipient réactionnel, Si la quantité d'un des premier et second composants immunologiques est insuffisante, il est possible de rajouter ce produit dans le même récipient réactionnel et de procéder après réaction à une nouvelle évaluation de OD. Ainsi, la solution technique préconisée selon l'invention permet de réaliser un dosage en retour "en un temps" (même microcuvette, mêmes réactifs, même milieu réactionnel, sans filtrations ni lavages intermédiaires).

Comme le réactif immunologique constitué par des particules de latex magnétiques sensibilisées par le premier composant immunologique et le réactif immunologique constitué par des particules non-magnétiques sensibilisées par le second composant immunologique sont chacun fournis en suspension dans un tampon biologique, on peut éviter de préparer spécialement un milieu tampon particulier pour la réaction immunologique d'agglutination selon le procédé de l'invention. Il suffit d'introduire l'échantillon contenant la substance immunologique à doser dans le milieu du premier réactif immunologique ou du second réactif immunologique avec lequel elle ne réagit pas, ou dans le mélange des milieux des deux si elle réagit avec chacun d'eux.

D'autres avantages et caractéristiques de l'invention seront mieux compris à la lecture qui va suivre d'exemples de réalisation nullement limitatifs mais donnés à titre d'illustration.

### *Exemple 1* Dosage du DDi

On met en contact au sein d'un tampon liquide disposé dans une microcuvette un échantillon de DDi, des particules de latex paramagnétiques ayant un diamètre d'environ 650 nm et sensibilisées par un anticorps monoclonal anti(DDi) (9C3) et des particules de latex non-magnétiques ayant un diamètre d'environ 300 nm et sensibilisées par un second anticorps monoclonal anti-DDi) (2F7) différent du premier anticorps monoclonal, le tampon liquide pouvant être ici le mélange des milieux de chaque anticorps.

On laisse réagir (i.e. incuber) pendant 0,25 h à RT. On crée un champ magnétique au moyen d'un aimant appliqué au niveau du fond extérieur de la microcuvette, puis maintient ce champ magnétique pendant la mesure de OD. Cette mesure est effectuée notamment par turbidimètrie à travers une portion transparente (P) de la paroi latérale de la microcuvette disposée en regard du surnageant.

Avec des échantillons renfermant des quantités croissantes de DDi, on a établi la courbe d'étalonnage de la figure 2 selon le diagramme OD en fonction de la concentration en DDi ; la concentration en DDi figure en abscisses (en µg/ml) et la OD mesurée à 450 ni est donnée en ordonnées.

La courbe de la figure 2 est une courbe exponentielle du type

$$Y = a + be^{-x/c} \qquad (1)$$

dans laquelle, l'expérimentation montre que l'on a pour le dosage du DDi :

a = 0,20557265
b = 0,97343085
c = 0,60269355

Avec la courbe d'étalonnage de la figure 2, l'on peut déterminer la teneur en DDi d'un échantillon à tester.

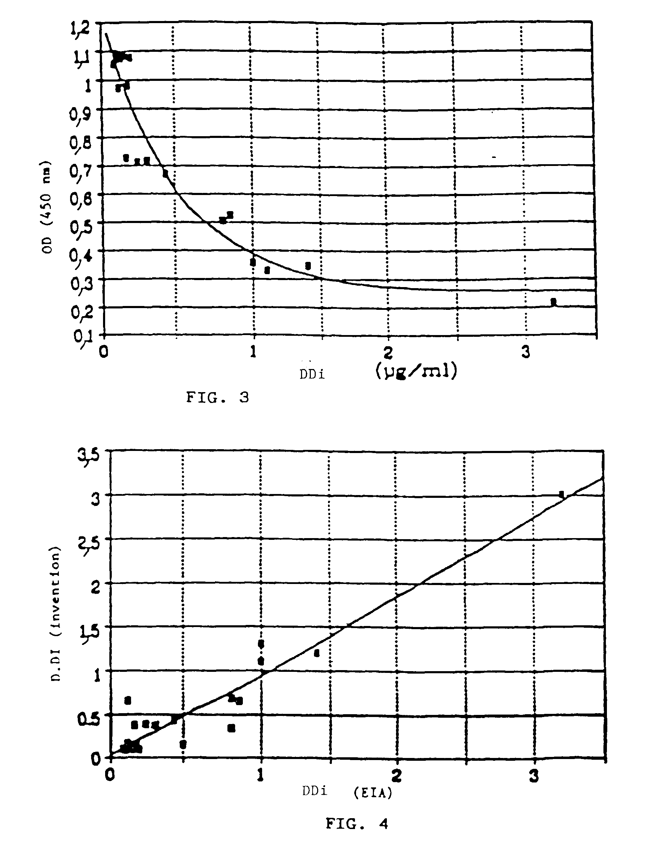

Pour comparaison, on a procédé à l'établissement de la courbe d'étalonnage de DDi par une technique EIA selon les réactifs et le protocole opératoire de la trousse "ASSERACHROM[R] D-DIMERE" (commercialisée par la société dite DIAGNOSTICA STAGO). La courbe obtenue est donnée dans la figure 3 selon le même système de coordonnées que la courbe de la figure 2. La courbe de la figure 3 est une courbe exponentielle selon l'équation (1) ci-dessus, avec

a = 0,25361282
b = 0,98641938
c = 0,49755979

La corrélation des résultats des mesures selon le procédé de dosage en retour de l'invention et le dosage en retour selon la technique EIA précitée est une droite comme représentée dans la figure 4. Le coefficient de corrélation ($r^2$) est supérieure à 0,9.

**Exemple 2** Dosage du PAI-1

On procède selon le protocole opératoire décrit à l'exemple 1 et illustré par la figure 1, au dosage en retour du PAI-1 contenu dans un échantillon au moyen d'un réactif immunologique constitué par des particules de latex paramagnétiques (diamètre : 600 nm) sensibilisées par un premier anticorps monoclonal anti(PAI-1) (7F5) et d'un réactif immunologique constitué par des particules de latex non-magnétiques (diamètre : 250 nm) sensibilisées par un second anticorps monoclonal anti(PAI-1) (7D4).

La courbe d'étalonnage du PAI-1 peut être représentée par une courbe exponentielle selon l'équation (1), ou une courbe hyperbolique sensiblement équivalente à ladite courbe exponentielle.

**Exemple 3** Dosage de la Protéine C

On procède selon le protocole opératoire décrit à l'exemple 1 et illustré par la figure 1, au dosage en retour de façon directe de la Protéine C, en utilisant deux anticorps monoclonaux spécifiques de la Protéine C, les particules de latex non-magnétiques étant fluorescentes.

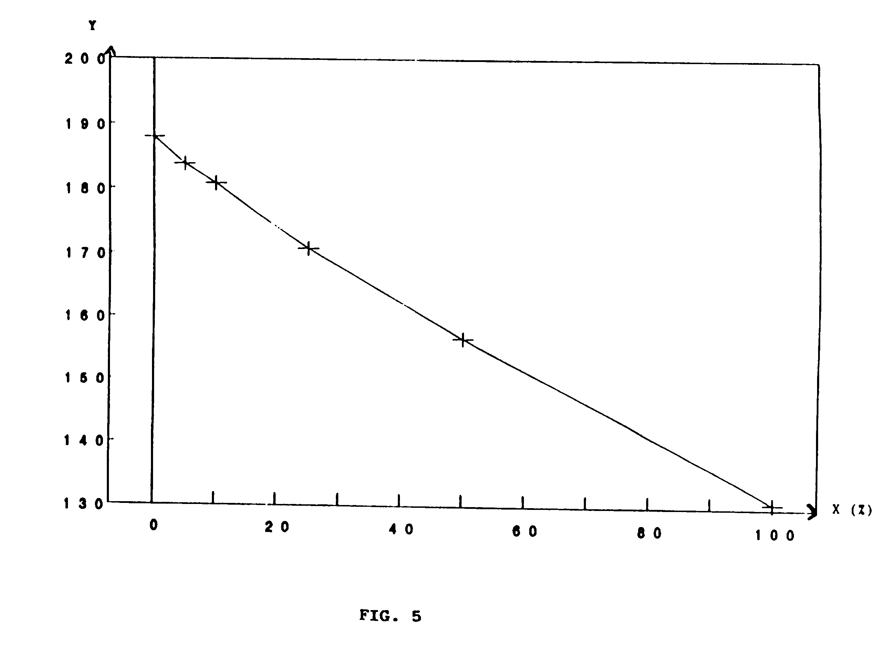

La courbe d'étalonnage correspondante selon le système fluorescence = fonction de la concentration en Protéine C est donnée à la figure 5 où X est la concentration en Protéine C (exprimée en % p/v) en abscisses, et Y est la fluorescence en ordonnées. Le seuil de sensibilité est de l'ordre de 10 ng/ml.

**Exemple 4** Dosage du DDi

On procède comme indiqué à l'exemple 1 ci-dessus, avec la différence que l'on effectue le dosage direct du DDi en utilisant des particules de latex paramagnétiques sensibilisées avec un anticorps anti(Ddi), à savoir ici l'anticorps 9C3, et des particules de latex fluorescent non-magnétiques sensibilisées avec DDi.

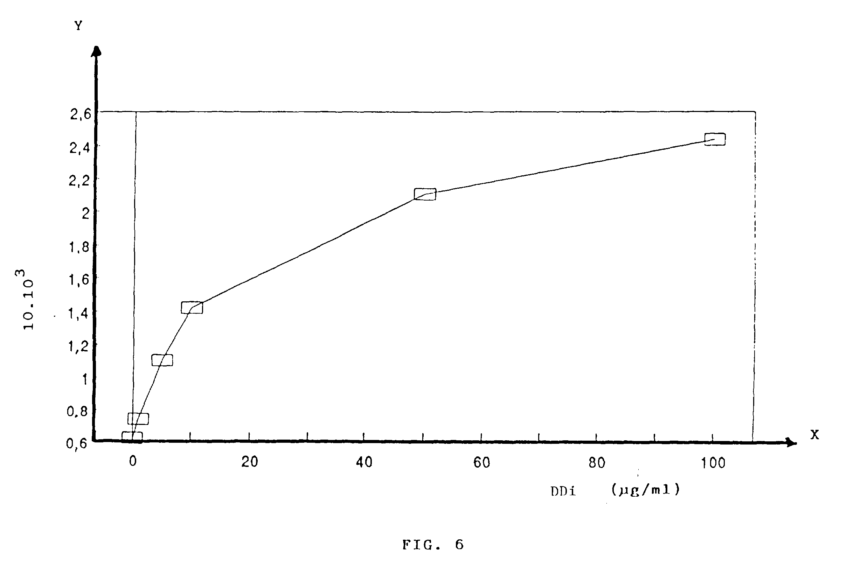

La courbe d'étalonnage correspondante selon le système fluorescence = fonction de la concentration en DDi libre est donnée à la figure 6 où X est la concentration en DDi libre (exprimée en μg/ml) en abscisses et Y est la fluorescence en ordonnées.

**Exemple 5** Dosage du PAI-1

On procède comme indiqué à l'exemple 2 ci-dessus, avec la différence que l'on effectue le dosage en retour du PAI-1 en utilisant des particules de latex paramagnétiques sensibilisées avec un anticorps anti (PAI-1), à savoir ici l'anticorps 7D4, et des particules de latex fluorescent non-magnétiques sensibilisées avec un second anticorps anti(PAI-1), à savoir ici l'anticorps 7F5.

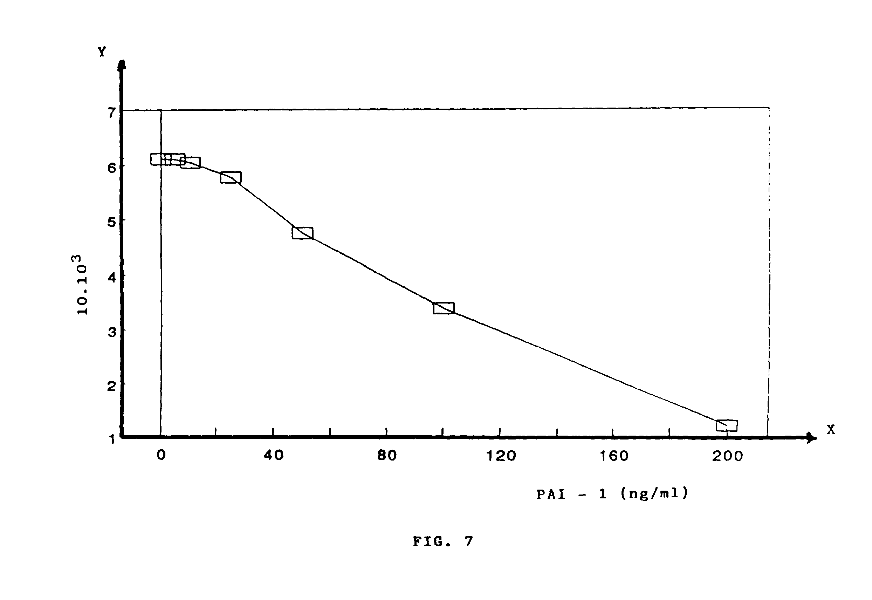

La courbe d'étalonnage correspondante selon le système fluorescence = fonction de la concentration en PAI-1 est donnée à la figure 7 où X est la concentration en PAI-1 (exprimée en ng/ml) en abscisses et Y est la fluorescence en ordonnées.

La sensibilité de ce dosage du PAI-1 est de l'ordre de 1 ng/ml.

**Exemple 6** Dosage de HBsAg

On procède selon le protocole opératoire décrit à l'exemple 1 et illustré par la figure 1, au dosage en retour de HBsAg, en utilisant deux anticorps monoclonaux (ou à la rigueur polyclonaux) spécifiques de HBsAg.

**Exemple 7** Dosage de HBsAb

On met en contact au sein d'un tampon liquide disposé dans une microcuvette un échantillon de HBsAb, des particules de latex paramagnétiques ayant un diamètre d'environ 650 nm et sensibilisées par un premier antigène HBsAg et des particules de latex non-magnétiques ayant un diamètre d'environ 300 nm et sensibilisées par un second antigène HBsAg différent du premier antigène, le tampon liquide pouvant être ici le mélange des milieux de chaque antigène.

On laisse réagir (i.e. incuber) pendant 0,25 h à RT. On crée un champ magnétique au moyen d'un aimant appliqué au niveau du fond extérieur de la microcuvette, puis maintient ce champ magnétique pendant la mesure de OD. Cette mesure est effectuée notamment par turbidimètrie à travers une portion transparente de la paroi latérale de la microcuvette disposée en regard du surnageant.

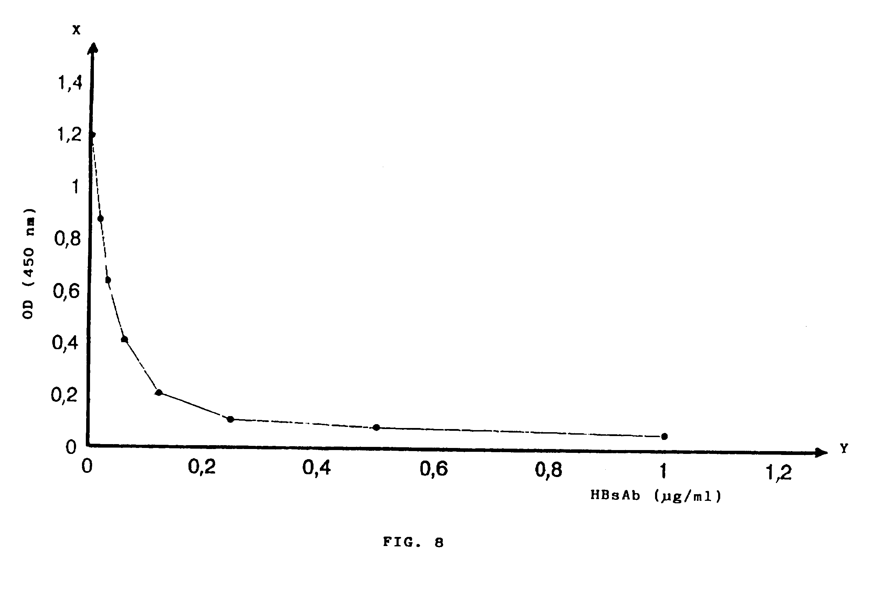

La courbe d'étalonnage correspondante obtenue par turbidimétrie selon le système OD = fonction de la concentration en HBsAb est donnée à la figure 8 où Y est la concentration en HBsAb (exprimée en μg/ml) en abscisses, et X est la OD en ordonnées.

En ce qui concerne le nécessaire de dosage ou d'identification, on préconise ce qui suit :

1°) quand ladite substance immunologique (X) est une substance antigénique, le premier réactif immunologique est sensibilisé par un anticorps monoclonal spécifique de X et le second réactif immunologique est sensibilisé par un anticorps monoclonal spécifique de X, différent du précédent et fixé sur des particules de latex non-magnétiques ;

2°) quand X est un anticorps, le premier réactif immunologique est sensibilisé par un antigène spécifique de X et le second réactif immunologique est sensibilisé par un antigène spécifique de X, différent du précédent et fixé sur des particules de latex non-magnétiques ;

3°) quand X est un antigène, le premier réactif immunologique est senbilisé par un anticorps monoclonal spécifique de X et le second réactif immunologique est sensibilisé par un anticorps monoclonal spécifique de X, différent du précédent et fixé sur des particules colloïdales d'or ou d'argent non-magnétiques ; et,

4°) quand X est un anticorps, le premier réactif immunologique est senbilisé par un antigène spécifique de X et le second réactif immunologique est sensibilisé par un antigène spécifique de X, différent du précédent et fixé sur des particules colloïdales d'or ou d'argent non-magnétiques.

***Essais comparatifs***

On a déterminé pour comparaison le seuil de sensibilité du dosage de HBsAb selon l'exemple 7 de l'invention avec la technique décrite dans US-A-4 115 535 précité mettant en oeuvre des particules de latex non-magnétiques sensibilisées par le premier antigène HBsAg et des particules de latex magnétiques et colorées sensibilisées par ledit premier antigène HBsAg, d'une part, et la technique décrite dans EP-A-0 410 893 précité mettant en oeuvre des particules de latex non-magnétiques sensibilisées par le premier antigène HBsAg et des particules de latex magnétiques et colorées sensibilisées par la protéine A.

Les résultats obtenus ont été consignés dans le tableau I ci-après. Ils montrent que la technique selon l'invention est bien plus sensible que celle des deux techniques antérieures.

TABLEAU 1

| *Seuil de sensibilité en HBsAb* | |
| --- | --- |
| TECHNIQUE | SEUIL DE SENSIBILITE |
| Ex. 7 selon l'invention | 0,04 μg/ml |
| US-A-4 115 535 | 0,60 μg/ml |
| EP-A-0 410 893 | 0,50 μg/ml |

**Revendications**

1. Procédé de dosage en retour de façon directe d'une substance immunologique (X) choisie parmi l'ensemble constitué par les antigènes et les anticorps et susceptible d'être présente dans un échantillon à tester, ledit procédé, qui met en oeuvre (i) des particules de latex magnétiques, (ii) des particules non-magnétiques et (iii) une réaction immunologique

$$X + antiX \rightarrow X\text{-}(antiX),$$

étant caractérisé en ce qu'il comprend les étapes consistant à

(1°) mettre en contact dans un milieu liquide ledit échantillon à tester et pouvant contenir ladite substance immunologique avec des quantités biologiquement connues

(A) d'un premier réactif immunologique en suspension dans un tampon et constitué par des particules de latex magnétiques sensibilisées par un premier composant qui est un anticorps (antiX) de ladite substance immunologique, et

(B) d'un second réactif immunologique en suspension dans un tampon et constitué par des particules non-magnétiques sensibilisées par un second composant qui est un anticorps de ladite substance immunologique, ledit second composant étant (i) stoechiométriquement en excès par rapport à ladite substance immunologique X à tester et (ni) différent dudit premier composant (antiX) ;

(2°) incuber le milieu réactionnel résultant pendant une durée inférieure ou égale à 1 h pour mettre en oeuvre la ou les réactions immunologiques X + antiX → X-(antiX) ;

(3°) appliquer un champ magnétique pour séparer, dans le milieu réactionnel résultant, les produits magnétiques déplacés par ledit champ magnétique des produits non-magnétiques non affectés par ledit champ magnétique ; puis, (4°) évaluer ladite substance immunologique (X), pouvant être présente dans l'échantillon à tester, par l'observation de la portion (P) du milieu réactionnel résultant ne contenant que des produits non-magnétiques en suspension dans ladite portion (P) et non affectés par la réaction immunologique et ledit champ magnétique.

2.  Procédé de dosage en retour de façon directe d'une substance immunologique (X) choisie parmi l'ensemble constitué par les anticorps et susceptible d'être présente dans un échantillon à tester, ledit procédé, qui met en oeuvre (i) des particules de latex magnétiques, (ii) des particules non-magnétiques et (iii) une réaction immunologique

Antigène + anticorps → antigène - anticorps

étant caractérisé en ce qu'il comprend les étapes consistant à

(1°) mettre en contact dans un milieu liquide ledit échantillon à tester et pouvant contenir ladite substance immunologique avec des quantités biologiquement connues

(A) d'un premier réactif immunologique en suspension dans un tampon et constitué par des particules de latex magnétiques sensibilisées par un premier composant qui est un antigène spécifique de ladite substance immunologique, et
(B) d'un second réactif immunologique en suspension dans un tampon et constitué par des particules non-magnétiques sensibilisées par un second composant qui est un antigène de ladite substance immunologique, ledit second composant étant (i) stoechiométriquement en excès par rapport à ladite substance immunologique X à tester et (ii) différent dudit premier composant antigène ;

(2°) incuber le milieu réactionnel résultant pendant une durée inférieure ou égale à 1 h pour mettre en oeuvre la ou les réactions immunologiques antigène + anticorps → antigène - anticorps ;
(3°) appliquer un champ magnétique pour séparer, dans le milieu réactionnel résultant, les produits magnétiques déplacés par ledit champ magnétique des produits non-magnétiques non affectés par ledit champ magnétique ; puis, (4°) évaluer ladite substance immunologique (X), pouvant être présente dans l'échantillon à tester, par l'observation de la portion (P) du milieu réactionnel résultant ne contenant que des produits non-magnétiques en suspension dans ladite portion (P) et non affectés par la réaction immunologique et ledit champ magnétique.

3.  Procédé de dosage d'une substance immunologique (X) choisie parmi l'ensemble constitué par les antigènes et les anticorps et susceptible d'être présente dans un échantillon à tester, ledit procédé, qui met en oeuvre (i) des particules de latex magnétiques, (ii) des particules non-magnétiques et (iii) une réaction immunologique

X + antiX → X-(antiX),

étant caractérisé en ce qu'il comprend les étapes consistant à

(1°) mettre en contact dans un milieu liquide ledit échantillon à tester et pouvant contenir ladite substance immunologique avec des quantités biologiquement connues

(A) d'un premier réactif immunologique en suspension dans un tampon et constitué par des particules de latex magnétiques sensibilisées par un premier composant qui est un anticorps (antiX) de ladite substance immunologique, et
(B) d'un second réactif immunologique en suspension dans un tampon et constitué par des particules non-magnétiques sensibilisées par un second composant qui est ladite substance immunologique ;

(2°) incuber le milieu réactionnel résultant pendant une durée inférieure ou égale à 1 h pour mettre en oeuvre la ou les réactions immunologiques X + antiX → X-(antiX) ;

(3°) appliquer un champ magnétique pour séparer, dans le milieu réactionnel résultant, les produits magnétiques déplacés par ledit champ magnétique des produits non-magnétiques non affectés par ledit champ magnétique ; puis, (4°) évaluer ladite substance immunologique (X), pouvant être présente dans l'échantillon à tester, par l'observation de la portion (P) du mi-

tester, par l'observation de la portion (P) du milieu réactionnel résultant ne contenant que des produits non-magnétiques en suspension dans ladite portion (P) et non affectés par la réaction immunologique et ledit champ magnétique.

4. Procédé suivant l'une quelconque des revendications 1, 2 ou 3, caractérisé en ce que la durée d'incubation du stade (2°), au cours de laquelle se développe l'ensemble des réactions immunologiques, est inférieure ou égale à 45 minutes, et mieux comprise entre 5 et 30 minutes.

5. Procédé suivant l'une quelconque des revendications 1, 2 ou 3, caractérisé en ce que les particules non-magnétiques sont des particules colloïdales d'or ou d'argent.

6. Procédé suivant l'une quelconque des revendications 1, 2 ou 3, caractérisé en ce que les particules non-magnétiques sont des particules de bentonite ou de charbon actif.

7. Procédé suivant la revendication 1, caractérisé en ce que (a) la substance immunologique à tester est un antigène, et (b) le premier réactif immunologique et le second réactif immunologique comportent chacun un anticorps spécifique de ladite substance immunologique.

8. Procédé suivant la revendication 3, caractérisé en ce que la substance immunologique X à tester est un anticorps, le premier réactif immunologique comporte un anticorps antiX spécifique de ladite substance immunologique X, ledit anticorps antiX étant un anti(anticorps), et le second réactif immunologique comporte un anticorps qui est ladite substance immunologique marquée.

9. Procédé suivant la revendication 1, caractérisé en ce que (a) la substance immunologique X à tester est un anticorps, et (b) le premier réactif immunologique et le second réactif immunologique comportent chacun un anticorps antiX spécifique de ladite substance immunologique X, chaque anticorps antiX étant un anti(anticorps)

10. Procédé suivant la revendication 1 ou 2, caractérisé en ce que l'évaluation de l'étape (4°) est effectuée par la mesure de la variation de densité optique (OD), ladite variation étant (i) proportionnelle à la concentration en particules non-magnétiques non affectées par le champ magnétique dans ladite portion (P) et (ni) inversement proportionnelle à la concentration en substance X à tester.

11. Procédé suivant l'une quelconque des revendications 1-3, caractérisé en ce que le composant du second réactif immunologique est fixé sur des particules de latex non-magnétiques.

12. Procédé suivant la revendication 1 ou 10, caractérisé en ce que l'évaluation de l'étape (4°) est effectuée, en présence du champ magnétique, par la détermination de la densité optique par transmission, diffusion, fluorescence, phosphorescence, luminescence ou chemiluminescence.

13. Nécessaire de dosage ou de détermination, selon le procédé de la revendication 1, 2 ou 3, d'une substance immunologique (X) pouvant être contenue dans un échantillon à tester, ledit nécessaire étant caractérisé en ce qu'il comprend

    - pour la mise en oeuvre du procédé selon la revendication 1 :

        un réactif immunologique constitué par des particules de latex magnétiques sensibilisées par un premier anticorps (antiX) de la substance X, et ;
        un réactif immunologique constitué par des particules non-magnétiques sensibilisées par un second anticorps de la substance X différent dudit premier anticorps antiX ;

    - pour la mise en oeuvre du procédé selon la revendication 2, où X est un anticorps :

        un réactif immunologique constitué par des particules de latex magnétiques sensibilisées par un premier antigène spécifique de l'anticorps X, et
        un réactif immunologique constitué par des particules non-magnétiques sensibilisées par un second antigène spécifique de l'anticorps X et différent dudit premier antigène ; et

    pour la mise en oeuvre du procédé selon la revendication 3 :

        un réactif immunologique constitué par des particules de latex magnétiques sensibilisées par un premier composant qui est un anticorps (antiX) de la substance X, et ;
        un réactif immunologique constitué par des particules non-magnétiques sensibilisées par un second composant qui est ladite substance X.

14. Nécessaire de dosage suivant la revendication 13 pour mettre en oeuvre le procédé selon la revendication 1, caractérisé en ce que, quand ladite substance immunologique X est un antigène, le premier réactif immunologique est sensibilisé par un anticorps monoclonal spécifique de la substance immunologique X à doser et le second réactif immunologique est sensibilisé par un anticorps monoclonal spécifique de ladite substance immunologique X à doser, différent du précédent et fixé sur des particules de latex non-magnétiques.

15. Nécessaire de dosage suivant la revendication 13 pour mettre en oeuvre le procédé selon la revendication 2, caractérisé en ce que, quand ladite substance immunologique X est un anticorps, le premier réactif immunologique est sensibilisé par un antigène spécifique de la substance immunologique X à doser et le second réactif immunologique est sensibilisé par un antigène spécifique de ladite substance immunologique X à doser, différent du précédent et fixé sur des particules de latex non-magnétiques.

16. Nécessaire de dosage suivant la revendication 13 pour mettre en oeuvre le procédé selon la revendication 1, caractérisé en ce que, quand ladite substance immunologique X est un antigène, le premier réactif immunologique est sensibilisé par un anticorps monoclonal spécifique de la substance immunologique X à doser et le second réactif immunologique est sensibilisé par un anticorps monoclonal spécifique de ladite substance immunologique X à doser, différent du précédent et fixé sur des particules colloïdales d'or ou d'argent non-magnétiques.

17. Nécessaire de dosage suivant la revendication 13 pour mettre en oeuvre le procédé selon la revendication 2, caractérisé en ce que, quand ladite substance immunologique X est un anticorps, le premier réactif immunologique est sensibilisé par un antigène spécifique de la substance immunologique X à doser et le second réactif immunologique est sensibilisé par un antigène spécifique de ladite substance immunologique X à doser, différent du précédent et fixé sur des particules colloïdales d'or ou d'argent non-magnétiques.

18. Nécessaire de dosage suivant la revendication 14 ou 15, caractérisé en ce que les particules de latex non-magnétiques sont colorées, fluorescentes, phosphorescentes, luminescentes ou chemiluminescentes.

**Patentansprüche**

1. Verfahren zur direkten Gegendosierung einer immunologischen Substanz (X), die aus der Gruppe ausgewählt ist, welche aus den Antigenen und den Antikörpern besteht, und geeignet ist, um in einer Testprobe vorzuliegen, wobei das Verfahren, das von (i) magnetischen Latexteilchen, (ii) nicht-magnetischen Teilchen und (iii) einer immunologischen Reaktion

$$X + AntiX \rightarrow X\text{-}(AntiX)$$

Gebrauch macht, dadurch gekennzeichnet ist, daß es die Schritte umfaßt, bestehend aus:

(1.) in Kontakt bringen der Testprobe, die die immunologische Substanz enthalten kann, in einer flüssigen Umgebung mit biologisch bekannten Mengen

(A) eines ersten immunologischen Reagens in Suspension in einem Tampon, das aus magnetischen Latexteilchen besteht, die durch einen ersten Bestandteil, der ein Antikörper (AntiX) der immunologischen Substanz ist, sensibilisiert sind, und
(B) eines zweiten immunologischen Reagens in Suspension in einem Tampon, das aus nicht-magnetischen Teilchen besteht, die durch einen zweiten Bestandteil, der ein Antikörper der immunologischen Substanz ist, sensibilisiert sind, wobei der zweite Bestandteil (i) im Verhältnis zur zu testenden immunologischen Substanz X stöchiometrisch im Überschuß vorliegt und (ii) vom ersten Bestandteil (AntiX) verschieden ist;

(2.) Inkubieren der resultierenden Reaktionsumgebung während einer Zeitdauer unterhalb oder gleich 1 h, um die immunologische(n) Reaktion(en)

$$X + AntiX \rightarrow X\text{-}(AntiX)$$

auszuführen;
(3.) Anlegen eines Magnetfeldes, um in der resultierenden Reaktionsumgebung die durch das Magnetfeld verlagerten magnetischen Produkte von den nicht-magnetischen Produkten, auf die durch das Magnetfeld nicht eingewirkt wird, zu trennen; und dann
(4.) Auswerten der immunologischen Substanz (X), die in der Testprobe vorliegen kann, durch die Beobachtung des Teils (P) der resultierenden Reaktionsumgebung, der nur nicht-magnetische Produkte enthält, die in dem Teil (P) in Suspension vorliegen und nicht von der immunologischen Reaktion und dem Magnetfeld betroffen sind.

2. Verfahren zur direkten Gegendosierung einer immunologischen Substanz (X), die aus der Gruppe ausgewählt ist, welche aus den Antikörpern besteht, und geeignet ist, um in einer Testprobe vorzuliegen, wobei das Verfahren, das von (i) magnetischen Latexteilchen, (ii) nicht-magnetischen Teilchen und (iii) einer immunologischen Reaktion

Antigen + Antikörper → Antigen - Antikörper

Gebrauch macht, dadurch gekennzeichnet ist, daß es die Schritte umfaßt, bestehend aus:

(1.) in Kontakt bringen der Testprobe, die die immunologische Substanz enthalten kann, in einer flüssigen Umgebung mit biologisch bekannten Mengen

(A) eines ersten immunologischen Reagens in Suspension in einem Tampon, das aus magnetischen Latexteilchen besteht, die durch einen ersten Bestandteil, der ein spezifisches Antigen der immunologischen Substanz ist, sensibilisiert sind, und (B) eines zweiten immunologischen Reagens in Suspension in einem Tampon, das aus nicht-magnetischen Teilchen besteht, die durch einen zweiten Bestandteil, der ein Antigen der immunologischen Substanz ist, sensibilisiert sind, wobei der zweite Bestandteil (i) im Verhältnis zur zu testenden immunologischen Substanz X stöchiometrisch im Überschuß vorliegt und (ii) vom ersten Antigen-Bestandteil verschieden ist;

(2.) Inkubieren der resultierenden Reaktionsumgebung während einer Zeitdauer unterhalb oder gleich 1 h, um die immunologische(n) Reaktion(en)

Antigen + Antikörper → Antigen - Antikörper

auszuführen;
(3.) Anlegen eines Magnetfeldes, um in der resultierenden Reaktionsumgebung die durch das Magnetfeld verlagerten magnetischen Produkte von den nicht-magnetischen Produkten, auf die durch das Magnetfeld nicht eingewirkt wird, zu trennen; und dann
(4.) Auswerten der immunologischen Substanz (X), die in der Testprobe vorliegen kann, durch die Beobachtung des Teils (P) der resultierenden Reaktionsumgebung, der nur nicht-magnetische Produkte enthält, die in dem Teil (P) in Suspension vorliegen und nicht von der immunologischen Reaktion und dem Magnetfeld betroffen sind.

3. Verfahren zur Dosierung einer immunologischen Substanz (X), die aus der Gruppe ausgewählt ist, welche aus den Antigenen und den Antikörpern besteht, und geeignet ist, um in einer Testprobe vorzuliegen, wobei das Verfahren, das von (i) magnetischen Latexteilchen, (ii) nicht-magnetischen Teilchen und (iii) einer immunologischen Reaktion

X + AntiX → X-(AntiX)

Gebrauch macht, dadurch gekennzeichnet ist, daß es die Schritte umfaßt, bestehend aus:

(1.) in Kontakt bringen der Testprobe, die die immunologische Substanz enthalten kann, in einer flüssigen Umgebung mit biologisch bekannten Mengen

(A) eines ersten immunologischen Reagens in Suspension in einem Tampon, das aus magnetischen Latexteilchen besteht, die durch einen ersten Bestandteil, der ein Antikörper (AntiX) der immunologischen Substanz ist, sensibilisiert sind, und (B) eines zweiten immunologischen Reagens in Suspension in einem Tampon, das aus nicht-magnetischen Teilchen besteht, die durch einen zweiten Bestandteil, der die immunologische Substanz ist, sensibilisiert sind;

(2.) Inkubieren der resultierenden Reaktionsumgebung während einer Zeitdauer unterhalb oder gleich 1 h, um die immunologische(n) Reaktion(en)

X + AntiX → X-(AntiX)

auszuführen;
(3.) Anlegen eines Magnetfeldes, um in der resultierenden Reaktionsumgebung die durch das Magnetfeld verlagerten magnetischen Produkte von den nicht-magnetischen Produkten, auf die durch das Magnetfeld nicht eingewirkt wird, zu trennen; und dann
(4.) Auswerten der immunologischen Substanz (X), die in der Testprobe vorliegen kann, durch die Beobachtung des Teils (P) der resultierenden Reaktionsumgebung, der nur nicht-magnetische Produkte enthält, die in dem Teil (P) in Suspension vorliegen und nicht von der immunologischen Reaktion und dem Magnetfeld betroffen sind.

4. Verfahren nach irgendeinem der Ansprüche 1, 2 oder 3, dadurch gekennzeichnet, daß die Dauer der Inkubation des Schritts (2.), in deren Verlauf sich die gesamten immunologischen Reaktionen entwickeln, geringer als oder gleich 45 Minuten ist und besser zwischen 5 und 30 Minuten liegt.

5. Verfahren nach irgendeinem der Ansprüche 1, 2 oder 3, dadurch gekennzeichnet, daß die nicht-magnetischen Teilchen kolloidale Gold- oder Silberteilchen sind.

6. Verfahren nach irgendeinem der Ansprüche 1, 2 oder 3, dadurch gekennzeichnet, daß die nicht-magnetischen Teilchen Bentonit- oder Aktivkohle-Teilchen sind.

7. Verfahren nach Anspruch 1, dadurch gekennzeichnet, daß (a) die zu testende immunologische Substanz ein Antigen ist und (b) das erste immunologische Reagens und das zweite immunologische Reagens jeweils einen spezifischen Antikörper der immunologischen Substanz enthalten.

8. Verfahren nach Anspruch 3, dadurch gekennzeichnet, daß die zu testende immunologische Substanz X ein Antikörper ist, das erste immunologische Reagens einen spezifischen Antikörper AntiX der immunologischen Substanz X enthält, wobei der Antikörper AntiX ein Anti(Antikörper) ist, und das zweite immunologische Reagens einen Antikörper enthält, der die markierte immunologische Substanz ist.

9. Verfahren nach Anspruch 1, dadurch gekennzeichnet, daß (a) die zu testende immunologische Substanz X ein Antikörper ist und (b) das erste immunologische Reagens und das zweite immunologische Reagens jeweils einen spezifischen Antikörper AntiX der immunologischen Substanz X enthalten, wobei jeder Antikörper AntiX ein Anti(Antikörper) ist.

10. Verfahren nach Anspruch 1 oder 2, dadurch gekennzeichnet, daß die Auswertung des Schritts (4.) durch die Messung der Änderung der optischen Dichte (OD) ausgeführt wird, wobei die Änderung (i) proportional zur Konzentration an nicht-magnetischen Teilchen, die nicht von dem Magnetfeld betroffen sind, in dem Teil (P) ist und (ii) invers proportional zur Konzentration der zu testenden Substanz X ist.

11. Verfahren nach irgendeinem der Ansprüche 1-3, dadurch gekennzeichnet, daß der Bestandteil des zweiten immunologischen Reagens an nicht-magnetische Latexteilchen gebunden ist.

12. Verfahren nach Anspruch 1 oder 10, dadurch gekennzeichnet, daß die Auswertung des Schritts (4.)

durch die Bestimmung der optischen Dichte durch Transmission, Diffusion, Fluoreszenz, Phosphoreszenz, Lumineszenz oder Chemilumineszenz in Gegenwart des Magnetfeldes ausgeführt wird.

13. Satz zur Dosierung oder zur Analyse einer immunologischen Substanz (X), die in einer Testprobe enthalten sein kann, gemäß dem Verfahren nach Anspruch 1, 2 oder 3, wobei der Satz dadurch gekennzeichnet ist, daß er umfaßt

- für die Ausführung des Verfahrens nach Anspruch 1:

  ein immunologisches Reagens, das aus magnetischen Latexteilchen besteht, die durch einen ersten Antikörper (AntiX) der Substanz X sensibilisiert sind, und ein immunologisches Reagens, das aus nicht-magnetischen Teilchen besteht, die durch einen zweiten Antikörper der Substanz X, der vom ersten Antikörper AntiX verschieden ist, sensibilisiert sind;

- für die Ausführung des Verfahrens nach Anspruch 2, bei dem X ein Antikörper ist:

  ein immunologisches Reagens, das aus magnetischen Latexteilchen besteht, die durch ein erstes spezifisches Antigen des Antikörpers X sensibilisiert sind, und ein immunologisches Reagens, das aus nicht-magnetischen Teilchen besteht, die durch ein zweites spezifisches Antigen des Antikörpers X sensibilisiert sind, das vom ersten Antigen verschieden ist; und

- für die Ausführung des Verfahrens nach Anspruch 3:

  ein immunologisches Reagens, das aus magnetischen Latexteilchen besteht, die durch einen ersten Bestandteil sensibilisiert sind, der ein Antikörper (AntiX) der Substanz X ist, und ein immunologisches Reagens, das aus nicht-magnetischen Teilchen besteht, die durch einen zweiten Bestandteil, der die Substanz X ist, sensibilisiert sind.

14. Satz zur Dosierung nach Anspruch 13 zur Ausführung des Verfahrens nach Anspruch 1, dadurch gekennzeichnet, daß, wenn die immunologische Substanz X ein Antigen ist, das erste immunologische Reagens durch einen spezifischen monoklonalen Antikörper der zu dosierenden immunologischen Substanz X sensibilisiert ist, und das zweite immunologische Reagens durch einen spezifischen monoklonalen Antikörper der zu dosierenden immunologischen Substanz X sensibilisiert ist, welcher vom vorhergehenden verschieden ist und an nicht-magnetische Latexteilchen gebunden ist.

15. Satz zur Dosierung nach Anspruch 13 zur Ausführung des Verfahrens nach Anspruch 2, dadurch gekennzeichnet, daß, wenn die immunologische Substanz X ein Antikörper ist, das erste immunologische Reagens durch ein spezifisches Antigen der zu dosierenden immunologischen Substanz X sensibilisiert ist, und das zweite immunologische Reagens durch ein spezifisches Antigen der zu dosierenden immunologischen Substanz X sensibilisiert ist, welches vom vorhergehenden verschieden ist und an nicht-magnetische Latexteilchen gebunden ist.

16. Satz zur Dosierung nach Anspruch 13 zur Ausführung des Verfahrens nach Anspruch 1, dadurch gekennzeichnet, daß, wenn die immunologische Substanz X ein Antigen ist, das erste immunologische Reagens durch einen spezifischen monoklonalen Antikörper der zu dosierenden immunologischen Substanz X sensibilisiert ist, und das zweite immunologische Reagens durch einen spezifischen monoklonalen Antikörper der zu dosierenden immunologischen Substanz X sensibilisiert ist, welcher vom vorhergehenden verschieden ist und an nicht-magnetische kolloidale Gold- oder Silberteilchen gebunden ist.

17. Satz zur Dosierung nach Anspruch 13 zur Ausführung des Verfahrens nach Anspruch 2, dadurch gekennzeichnet, daß, wenn die immunologische Substanz X ein Antikörper ist, das erste immunologische Reagens durch ein spezifisches Antigen der zu dosierenden immunologischen Substanz X sensibilisiert ist, und das zweite immunologische Reagens durch ein spezifisches Antigen der zu dosierenden immunologischen Substanz X sensibilisiert ist, welches vom vorhergehenden verschieden ist und an nicht-magnetische kolloidale Gold- oder Silberteilchen gebunden ist.

18. Satz zur Dosierung nach Anspruch 14 oder 15, dadurch gekennzeichnet, daß die nicht-magnetischen Latexteilchen gefärbt sind, fluoreszieren, phosphoreszieren, lumineszieren oder chemilumineszieren.

**Claims**

1. A method of assaying, by a direct back assay, an immunological substance (X) selected from the group consisting of antigens and antibodies and which may be present in a sample to be tested, said method, which uses (i) magnetic latex particles, (ii) non-magnetic particles and (iii) an immunological reaction :

   antigen + antibody → antigen-antibody,

   said method comprising the steps consisting in :

   (1°) bringing into contact in a liquid medium said sample to be tested and susceptible to contain said immunological substance with biologically known quantities of

   (A) a first immunological reagent in suspension in a buffer and consisting of magnetic latex particles sensitized by a first immunological product, which is an antibody (antiX) raised against said immunological substance, and
   (B) a second immunological reagent in suspension in a buffer and consisting of non-magnetic particles sensitized by a second immunological product, which is an antibody raised against said immunological substance, said second immunological reagent being (i) stoichiometrically in excess vis-a-vis said substance X to be tested and (ii) different from said first immunological reagent (antiX) ;

   (2°) incubating the resulting reaction medium for a period lower than or equal to 1 h in order to perform the immunological reaction(s)

   X + antiX → X - (antiX);

   (3°) applying a magnetic field to separate, in the resulting reaction medium, the magnetic products displaced by said magnetic field from the non-magnetic products unaffected by said magnetic field ; then
   (4°) evaluating said immunological substance (X), susceptible to be present in the sample to be tested, by the observation of a portion (P) of the resulting reaction medium containing only non-magnetic products in suspension in said portion P and unaffected by said immunological reaction and said magnetic field.

2. A method of assaying, by a direct back assay, an immunological substance (X) selected from the group consisting of antibodies and which may be present in a sample to be tested, said method, which uses (i) magnetic latex particles, (ii) non-magnetic particles and (iii) an immunological reaction :

antigen + antibody → antigen-antibody

said method comprising the steps consisting in :

(1°) bringing into contact in a liquid medium said sample to be tested and susceptible to contain said immunological substance with biologically known quantities of

    (A) a first immunological reagent in suspension in a buffer and consisting of magnetic latex particles sensitized by a first immunological product, which is an antigen specific of said immunological substance, and

    (B) a second immunological reagent in suspension in a buffer and consisting of non-magnetic particles sensitized by a second immunological product, which is an antigen specific of said immunological substance, said second immunological reagent being (i) stoichiometrically in excess vis-a-vis said substance X to be tested and (ii) different from said first immunological antigen reagent ;

(2°) incubating the resulting reaction medium for a period lower than or equal to 1 h in order to perform said immunological reaction(s)

antigen + antibody → antigen-antibody ;

(3°) applying a magnetic field to separate, in the resulting reaction medium, the magnetic products displaced by said magnetic field from the non-magnetic products unaffected by said magnetic field ; then,
(4°) evaluating said immunological substance (X), susceptible to be present in the sample to be tested, by the observation of a portion (P) of the resulting reaction medium containing only non-magnetic products in suspension in said portion (P) and unaffected by said immunological reaction and said magnetic field.

3. A method of assaying an immunological substance (X) selected from the group consisting of antigens and antibodies and which may be present in a sample to be tested, said method, which uses (i) magnetic particles, (ii) non-magnetic latex particles and (iii) an immunological reaction :

$$X + antiX \rightarrow X - (antiX) ;$$

said method comprising the steps consisting in :

(1°) bringing into contact in a liquid medium said sample to be tested and susceptible to contain said immunological substance with biologically known quantities of

    (A) a first immunological reagent in suspension in a buffer and consisting of magnetic latex particles sensitized by a first immunological product, which is an antibody (antiX) raised against said immunological substance, and
    (B) a second immunological reagent in suspension in a buffer and consisting of non-magnetic particles sensitized by a second immunological product, which is said immunological substance ;

(2°) incubating the resulting reaction medium for a period lower than or equal to 1 h in order to perform said immunological reaction(s)

$$X + antiX \rightarrow X - (antiX) ;$$

(3°) applying a magnetic field to separate, in the resulting reaction medium, the magnetic products displaced by said magnetic field from the non-magnetic products unaffected by said magnetic field ; then,
(4°) evaluating said immunological substance (X), susceptible to be present in the sample to be tested, by the observation of a portion (P) of the resulting reaction medium containing only non-magnetic products in suspension in said portion (P) and unaffected by said immunological reaction and said magnetic field.

4. A method according to claim 1, 2 or 3, wherein the incubation of step (2°), during which all the immunological reactions develop, has a duration lower than or equal to 45 minutes and preferably in the range between 5 and 30 minutes.

5. A method according to claim 1, 2 or 3, wherein the non-magnetic particles are colloidal particles of gold, or colloidal particles of silver, particles of bentonite, particles of activated charcoal or latex particles.

6. A method according to claim 1, 2 or 3, wherein the non-magnetic particles are particles of bentonite or activated charcoal.

7. A method according to claim 1, wherein (a) the immunological substance to be tested is an antigen, and (b) the first immunological reagent and the second immunological reagent comprise each a specific antibody of said immunological substance.

8. A method according to claim 3, wherein the immunological substance X to be tested is an antibody, the first immunological reagent comprises a specific antibody (antiX) of said immunological substance X, said antiX antibody being an anti(antibody), and the second immunological reagent which is an antibody consisting of tag-containing X.

9. A method according to claim 1, wherein (a) the immunological substance X to be tested is an antibody, and (b) the first immunlogical reagent and the second immunological reagent comprise each a specific antibody (antiX) of said immunological substance (X), each antiX antibody being an anti(antibody).

10. A method according to claim 1 or 2, wherein the evaluation of step (4°) is performed by measuring a variation in optical density (OD), said variation being (i) proportional to the concentration of non-magnetic particles in said portion (P) which are unaffected by the magnetic field, and (ii) inversely proportional to the concentration of said substance X to be tested.

11. A method according to claim 1, 2 or 3, wherein the second immunological reagent is bound to non-magnetic latex particles.

12. A method according to claim 1 or 10, wherein the evaluation of step (4°) is performed, in the presence of the magnetic field, by the determination of the optical density by transmission, diffusion, fluorescence, phosphorescence, luminescence or chemiluminescence.

13. A kit to assay or determine, according to the method of claim 1, 2 or 3, an immunological substance (X) susceptible to be present in a sample to be tested, said kit being characterized in that it comprises

   - for carrying out the method of claim 1 :

      an immunological reagent consisting of magnetic latex particles sensitized by a first antibody (antiX) raised against said immunological substance X, and an immunological reagent consisting of non-magnetic particles sensitized by a second antibody raised against said substance X, and different from said first antiX antibody ;

   - for carrying out the method of claim 2, wherein X is an antibody :

      an immunological reagent consisting of magnetic latex particles sensitized by a first antigen specific of said immunological substance X, and an immunological reagent consisting of non-magnetic particles sensitized by a second antigen specific of said immunological substance X, and different from said first antigen ; and,

   - for carrying out the method of claim 3 :

      an immunological reagent consisting of magnetic latex particles sensitized by a first antibody (antiX) raised against said immunological substance X, and an immunological reagent consisting of non-magnetic particles sensitized by a second immunological product, which is said substance X.

14. A kit according to claim 13 for carrying out the method of claim 1, wherein, when said immunological substance X is an antigen, the first immunological reagent is sensitized by a specific monoclonal antibody (antiX) of the immunological substance X to be assayed, and the second immunological reagent is sensitized by a specific monoclonal antibody of said immunological substance X to be assayed, which is different from the preceding one and is bound to non-magnetic latex particles.

15. A kit according to claim 13 for carrying out the method of claim 2, wherein, when said immunological substance X is an antibody, the first immunological reagent is sensitized by a specific antigen of the immunological substance X to be assayed and the second immunological reagent is sensitized by a specific antigen of said immunological substance X to be assayed, which is different from the preceding one and is bound to non-magnetic latex particles.

16. A kit according to claim 13 for carrying out the method of claim 1, wherein, when said immunological substance X is an antigen, the first immunological reagent is sensitized by a specific monoclonal antibody of the immunological substance X to be assayed, and the second immunological reagent is sensitized by a specific monoclonal antibody of said immunological substance X to be assayed, which is different from the preceding one and bound to non-magnetic particles of colloidal gold or silver.

17. A kit according to claim 13 for carrying out the method of claim 2, wherein, when said immunological substance X is an antibody, the first immunological reagent is sensitized by a specific antigen of the immunological substance X to be assayed, and the second immunological reagent is sensitized by a specific antigen of said immunological substance X to be assayed, which is different from the preceding one and bound to non-magnetic particles of colloidal gold or silver.

18. A kit according to claim 14 or 15, wherein the non-magnetic latex particles are colored, fluorescent, phosphorescent, luminescent or chemiluminescent.

FIG. 1

FIG. 2

FIG. 3

FIG. 4

22

FIG. 5

FIG. 6

EP 0 711 414 B1

FIG. 7

FIG. 8